# EUROPEAN PATENT APPLICATION

(11) **EP 1 736 550 A1**
(43) Date of publication of application: **27.12.2006**
(21) Application number: 05425452.9
(22) Date of filing: 22.06.2005
(51) Int. Cl.: C12P 13/02, C12P 17/04, C12P 9/00, C07D 307/87

(54) **Chemoenzymatic process for the synthesis of escitalopram**

(71) Applicant: Adorkem Technology SpA, 24062 Costa Volpino (Bergamo) (IT)
(72) Inventor: Cotticelli, Giovanni, Cernusco S/N (MI) (IT); Salvetti, Raul, 25040 Malonno (BS) (IT); Bertoni, Chiara, 24060 Rogno (BG) (IT)
(74) Representative: Pistolesi, Roberto

(57) **Abstract**

A process is described for the preparation of escitalopram and the pharmaceutically acceptable salts thereof starting from 5-cyanophthalide by a process which provides an enantioselective enzymatic deacylation reaction of a complex of the formula 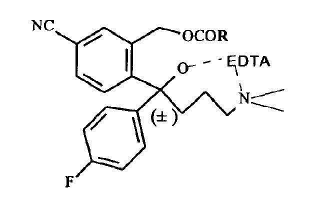 where R represents a C₁-C₄ alkyl residue or an aryl residue under the action of an esterase from *Aspergillus niger.*

## Description

The present invention relates to a process for the preparation of (+)-(3-dimethylaminopropyl)-1-(4'-fluorophenyl)-1,3-dihydroisobenzofuran-5-carbonitrile, which is better known under the name of escitalopram.

### PRIOR ART

The above-stated compound, the structural formula of which is given below, 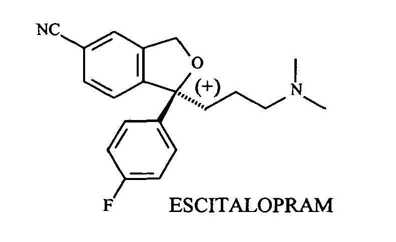 is the S(+) enantiomer of "citalopram", an active ingredient used for the preparation of pharmaceutical compositions intended for the treatment of depression.

Citalopram was first described in Belgian patent application BE850401 (and in the corresponding United States patent US 4,136,193); many patent documents furthermore relate to methods for the preparation thereof.

Since it has a chiral centre, citalopram was originally produced and marketed in the form of a racemic mixture.

However, as stated in EP347066, the S(+) enantiomer, better known as escitalopram, is responsible for virtually the entirety of the pharmacological activity of racemic citalopram. European patent application EP347066 describes substantially two methods for the preparation of escitalopram.

The first provides starting from racemic 4-(4-dimethylamino)-1-(4'-fluorophenyl)-1-(hydroxybutyl)-3-(hydroxymethyl)benzonitrile, which is subsequently esterified with an enantiomerically active acyl chloride such as (+) or (-)-α-methoxy-α-trifluoromethylphenylacetyl chloride. Two diastereoisomeric esters are obtained from each acyl chloride which are separated by HPLC, resulting in an enantiomerically pure ester; subsequent cyclisation in the presence of potassium tert.-butoxide in toluene allows the pure enantiomer citalopram to be obtained from each ester.

The second provides starting from enantiomerically pure (for example (+)) 4-(4-dimethylamino)-1-(4'-fluorophenyl)-1-(hydroxybutyl)-3 -(hydroxymethyl)-benzonitrile. In order to obtain said enantiomerically pure product, the amine is salified with an enantiomerically active acid, such as for example tartaric acid to yield two diastereoisomeric salts which can be separated by crystallisation. The pure enantiomer, unblocked from the salt thereof, is esterified to yield a particularly labile ester (for example with methanesulfonyl chloride) which, with the use of strong organic bases (for example triethylamine), makes it possible to obtain enantiomerically pure citalopram.

Other methods for preparing escitalopram are for example described in United States patent US6,365,747, in United States patent application US2003/0060641, and in International patent applications WO03/000672, WO03/006449, WO03/051861 and W02004/065375.

The above-described methods are, however, characterised by the use of enantiomerically active acids and/or diastereoisomeric separations by crystallisation or by the use of "simulated moving bed'' (SMB) methods which set limits with regards to scalability of the process, reaction yields and industrial applicability.

### DESCRIPTION OF THE INVENTION

International patent application PCT/IB2005/001067, filed on 7 April 2005 in the name of the present applicant, claiming priority from Italian application MI2004A000717, of 9 April 2004, describes an enzymatic resolution process under the action of an esterase from *Aspergillus niger* for the racemic mixture of a compound of the formula I 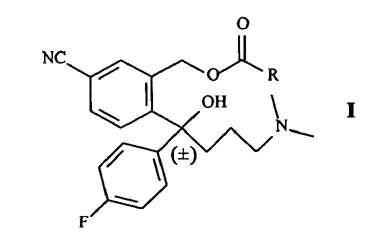 where R represents a C₁-C₄ alkyl residue or an aryl residue to yield the corresponding (-) enantiomer of the formula II.

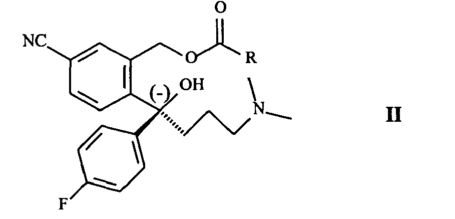

The (-) enantiomer obtained in this manner is then converted by hydrolysis into (-)4-(4-dimethylamino)-1-(4'-fluorophenyl)-1-(hydroxybutyl)-3-(hydroxymethyl)-benzonitrile of the formula III.

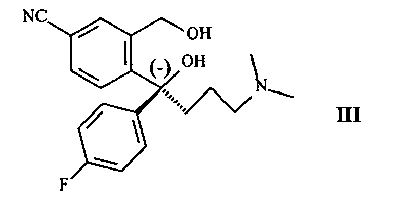

The latter is then converted into escitalopram by condensing the two hydroxyl groups using known prior art methods, such as for example the method described in EP347066.

A novel process which improves on that described in the above-stated international patent application PCT/IB2005/001067 has now been found which makes it possible to prepare escitalopram with greater enantiomeric purity.

The process according to the present invention provides that the enzymatic resolution under the action of an esterase, such as for example that from *Aspergillus niger,* is no longer carried out on the previously stated compound of the formula I but instead on a complex with EDTA of the formula IV shown below, 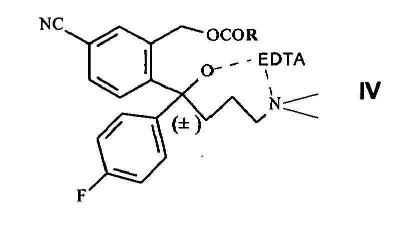 where R represents a C₁-C₄ alkyl residue or an aryl residue.

According to the better embodiment of the invention, R is a methyl residue; the corresponding complex with EDTA is thus 4-(4-dimethylamino)-1-(4'-fluorophenyl)-1-(butoxydiethylammonium tetracetic)-3-(acetoxymethyl)benzonitrile.

The complex in question may be obtained by reacting 5-cyanophthalide with a mixture of 4-fluorophenylmagnesium bromide and dimethylaminopropylmagnesium chloride to yield the compound 4-(4-dimethylamino)-1-(4'-fluorophenyl)-1-(butyloxymagnesium halide)-3-(hydroxymethyl)benzonitrile (V)

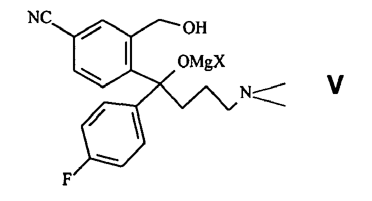

Said process is described in European patent application EP1506963, in the name of the present applicant. The compound (II) is then subsequently reacted with an acyl donor according to known prior art methods, for example by reaction with the anhydride or chloride of the corresponding acid. It is preferably reacted with acetyl chloride or with acetic anhydride to yield the corresponding 4-(4-dimethylamino)-1-(4'-fluorophenyl)-1-(hydroxybutyl)-3-(acetoxymethyl)benzonitrile.

The compound obtained in this manner is then dissolved in a mixture of alcohol and water, in the ratios specified below, in the presence of EDTA or of a salt thereof, such as for example the disodium or potassium salt. The EDTA is preferably used in slight excess relative to the substrate, preferably in an amount of between 1 and 1.5 equivalents, preferably between 1.2 and 1.3 equivalents.

Alternatively, the complex with EDTA may be obtained by reacting a diol of the formula VI, stated below, first with the above-stated acyl donor and then with

EDTA or with a salt thereof.

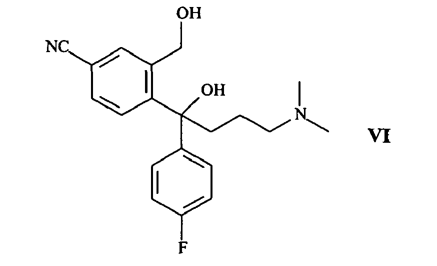

Said diol may in turn be obtained according to the method described in European patent application EP-171943. In greater detail, said method provides two consecutive Grignard reactions starting from 5-cyanophthalide, the first with 4-fluorophenylmagnesium bromide and the second, on the magnesium derivative obtained in this manner (formula VII), 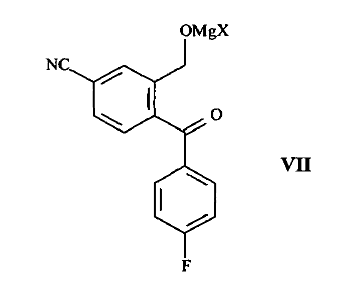 with 3-(dimethylamino)propylmagnesium chloride to obtain firstly a magnesium intermediate (formula VIII) 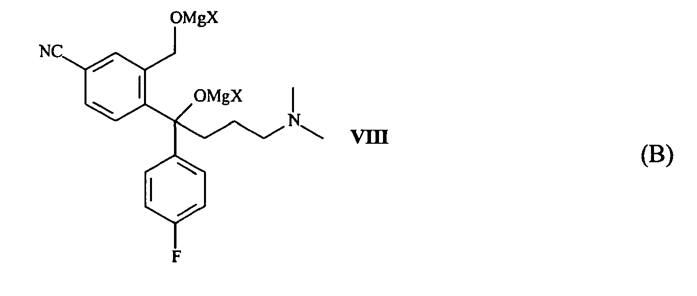 which, following acid hydrolysis, is converted into the diol of the formula VI.

The complex with EDTA in which R is methyl, namely 4-(4-dimethylamino)-1-(4'-fluorophenyl)-1-(butoxydiethylammonium tetracetic)-3-(acetoxymethyl)benzonitrile, was structurally characterised by ¹H- NMR analysis using a Bruker AMX 400 MHz spectrometer, as stated below.

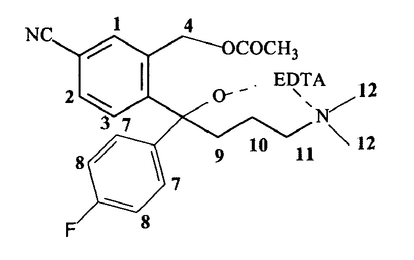

| ¹H-NMR (D₂O) | | | |
|---|---|---|---|
| δ(ppm) | Multiplicity | No. H | Attribution |
| 7.94-7.90 | d | 1 | H2 |
| 7.87-7.85 | d | 1 | H3 |
| 7.74 | s | 1 | H1 |
| 7.27-7.24 | d | 2 | H7 |
| 7.14-7.11-7.09 | d | 2 | H8 |
| 4.79-4.76 | m | 2 | H4 |
| 3.7 | s | 8H | (CH2-COO) EDTA |
| 3.5 | s | 4H | (N-CH2) EDTA |
| 2.91-2.82 | m | 2 | H11 |
| 2.58 | s | 6 | H12 |
| 2.31-2.22 | m | 2 | H9 |
| 1.95 | s | 3 | H5 |
| 1.62-135 | 2m | 2 | H10 |

The complex with EDTA is subjected to an enzymatic deacylation reaction under the action of an esterase, preferably from *Aspergillus niger,* and to subsequent treatment in a basic environment, preferably at a pH of between 7 and 9.5, to yield the corresponding (-) enantiomer of the formula II.

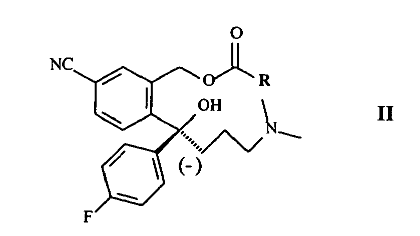

The resolution phase is advantageously performed in a solvent made up of a mixture of an alcohol (preferably a C₁-C₄ alcohol, still more preferably ethanol) and water, preferably in a ratio by volume of between 0.2-1.2 of aqueous phase and 1.0-1.5 of alcoholic phase, still more preferably 0.7 of aqueous phase and 1.3 of alcoholic phase, working at a preferential temperature of between 15 and 35°C, preferably between 20 and 25°C.

The water is advantageously used in the form of a phosphate buffer, preferentially monobasic potassium phosphate buffer.

The solvent is advantageously used in an amount of 3-5 litres, preferably 3.5- 4 litres, per mole of substrate.

According to a preferred aspect, the racemic compound of the formula I is initially added to the solvent at a basic pH value, preferentially of around 8, and subsequently adjusted to 6.

The esterase enzyme, preferably from *Aspergillus niger* is then added; the esterase is preferably immobilised on resins, generally epoxy resins (Eupergit C), and is advantageously used in an amount of 2500-3200 units, preferably 2800-2900 units, per mole of substrate.

The resolution reaction may be monitored by HPLC; on completion, after filtration of the enzymatic derivative, an extraction is performed using an aprotic polar solvent, such as for example ethyl acetate, as the preferential solvent, working at a pH of between 7 and 9.5; after subsequent evaporation and optional crystallisation, the (-) enantiomer of the formula II is obtained.

The (-) enantiomer II obtained in this manner may then be converted by means of hydrolysis, preferably basic hydrolysis, into the (-)4-(4-dimethylamino)-1-(4'-fluorophenyl)-1-(hydroxybutyl)-3-(hydroxymethyl)-benzonitrile of the formula III.

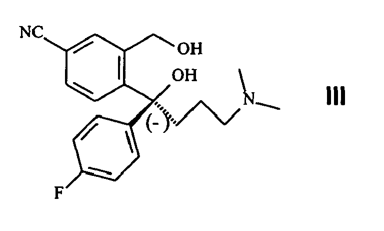

This hydrolysis reaction is described in the above-mentioned international patent application PCT/IB2005/001067.

The compound of the formula III may then in turn be converted into escitalopram by condensing the two hydroxyl groups using known prior art methods, such as for example the method described in EP347066 or the method described in Italian patent application MI2004A001872 filed by the present applicant on 1 October 2004, in which triphenylphosphine and ethyl azodicarboxylate are used in a configuration inversion reaction to yield the final escitalopram.

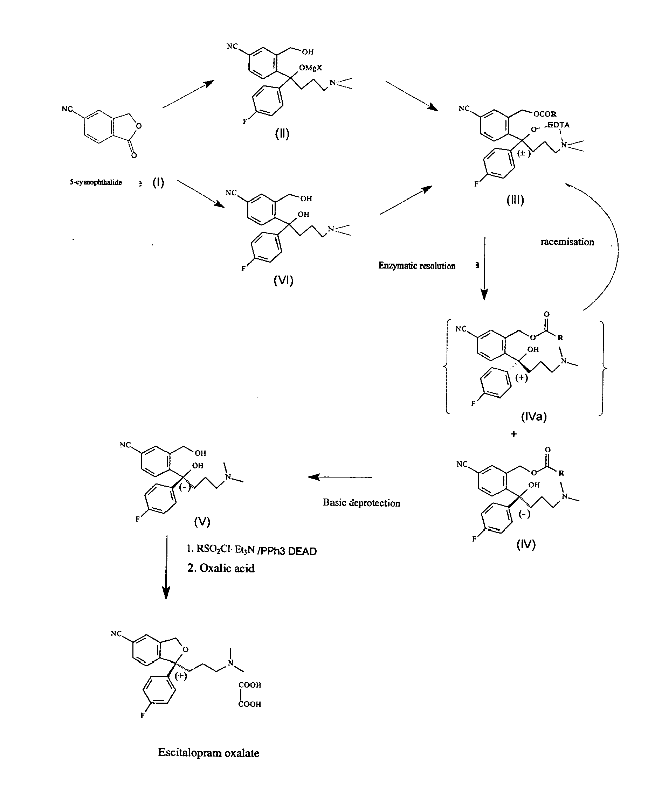

The complete reaction scheme is shown in Figure 1.

It has furthermore surprisingly been found that the (+) enantiomer (IV), namely (+)4-(4-dimethylamino)-1-(4'-fluorophenyl)-1-(hydroxybutyl)-3-(acyloxymethyl)benzonitrile, which is not capable of enzymatic hydrolysis, and which thus remains as a secondary product of the reaction with the esterase, may readily be transformed by means of a racemisation reaction in an acidic environment, preferably by hydrochloric acid, still more preferably at a pH of between 0.5 and 4, into the corresponding racemic (±)4-(4-dimethylamino)-1-(4'-fluorophenyl)-1-(hydroxybutyl)-3-(hydroxymethyl)benzonitrile, which, after acylation, may in turn be recirculated into the reaction with the esterase, so enhancing the overall process yield of the escitalopram synthesis.

For the purposes of the present invention, the terms "racemic mixture", "racemer", "racemic compound" are intended to mean not only a 50:50 mixture of the two individual enantiomers, but also a mixture in which one of the two enantiomers is present in excess relative to the remaining enantiomer.

The term EDTA is used here, in accordance with current practice, to denote ethylenediaminetetraacetic acid.

All references contained in the "description of the invention" should be deemed to be incorporated herein by reference to the extent that they may contain features usable for implementing the present invention.

The following Examples are purely illustrative and should not be considered to limit the invention.

### Example 1: Synthesis of 4-(4-dimethylamino)-1-(4'-fluorophenyl)-1-(hydroxybutyl)-3-(hydroxymethyl)benzonitrile

100 g of 5-cyanophthalide and 1.5 litres of tetrahydrofuran are introduced into a 10 litre reactor under an inert nitrogen atmosphere. 1100 g of 4-fluorophenylmagnesium bromide are added dropwise with stirring at a temperature of approx. -10°C. Once addition of the first Grignard reagent is complete, 467 g of dimethylaminopropylmagnesium chloride are added dropwise.

The reaction is terminated by slow dropwise addition of 1.2 litres of a 15 wt.% aqueous solution of ammonium chloride. The phases are separated. The upper phase is concentrated under a vacuum until a residue is obtained. An oil is obtained which is resuspended with 1 litre of toluene and 1 litre of demineralised water. The phases are separated, the upper phase is concentrated until a residue is obtained. 223.5 g of oil are obtained, which correspond to 172 g of 4-(4-dimethylamino)-1-(4'-fluorophenyl)-1-(hydroxybutyl)-3-(hydroxymethyl)benzonitrile.

### Example 2: Synthesis 4-(4-dimethylamino)-1-(4'-fluorophenyl)-1-(butyloxymagnesium halide)-3-(hydroxymethyl)benzonitrile and 4-(4-dimethylamino)-1-(4'-fluorophenyl)-1-(hdyroxybutyl)-3-(hydroxymethyl)benzonitrile

A mixture made up of 1150 g (1.15 moles) of a 20 wt.% solution in THF of 4-fluorophenylmagnesium bromide and 338 g (0.69 moles) of a 30 wt.% solution in THF of dimethylaminopropylmagnesium chloride is prepared at ambient temperature. The initially prepared mixture is added in approx. 2 hours to a suspension of 100 g (0.63 moles) of 5-cyanophthalide in 750 ml of tetrahydrofuran under a nitrogen atmosphere at -10 ÷ 0°C. Once addition is complete (see note 1), 1.2 litres of a 15 wt.% aqueous ammonium chloride solution are added dropwise.

The phases are separated. The upper phase is concentrated under a vacuum until a residue is obtained. An oil is obtained which is resuspended with 1 litre of toluene and 1 litre of demineralised water. The phases are separated, the upper phase is concentrated until a residue is obtained. 232 g of oil are obtained, which correspond to 181 g of 4-(4-dimethylamino)-1-(4'-fluorophenyl)-1-(hydroxybutyl)-3-(hydroxymethyl)benzonitrile.

### Example 3: Synthesis of 4-(4-dimethylamino)-1-(4'-fluorophenyl)-1-(butoxydiethylammonium tetracetic)-3-(acetoxymethyl)benzonitrile and enzymatic resolution to yield (-)4-(4-dimethylamino)-1-(4'-fluorophenyl)-1-(hydroxybutyl)-3-(hydroxymethyl)benzonitrile hydrochloride

172 g of 4-(4-dimethylamino)-1-(4'-fluorophenyl)-1-(hydroxybutyl)-3-(hydroxymethyl)benzonitrile and 670 ml of dichloromethane are introduced into a 2 litre four-necked flask. 146.3 g of acetyl chloride are added dropwise at a temperature of approx. 35-40°C. The mixture is concentrated under a vacuum until a residue is obtained. 256.9 g of 4-(4-dimethylamino)-1-(4'-fluorophenyl)-1-(hydroxybutyl)-3-(acetoxymethyl)benzonitrile hydrochloride are obtained in the form of an orange oily residue.

¹H NMR (DMSO-d6) δ 7.9 (d, 1H), 7.8 (d, 1H), 7.75 (s, 1H), 7.2 (d, 2H), 7.1 (d, 2H) 6.2 (s, 1H), 5.2 (d, 1H), 4.8 (d, 1H), 3.0 (m, 2H), 2.60 (m, 6H), 2.3 (s, 2H), 1.9 (s, 3H), 1.7 (m, 1H), 1.4 (m, 1H).2

The oil is resuspended with 2.5 litres of EtOH until solubilisation is complete; 2.5 litres of water and 210 g of disodium EDTA are added.

The mixture is partially evaporated until crystallisation occurs and a solid is obtained. ¹H NMR (DMSO-d6) δ 7.9 (d, 1H), 7.8 (d, 1H), 7.75 (s, 1H), 7.2 (d, 2H), 7.1 (d, 2H) 5.2 (d, 1H), 4.8 (d, 1H), 3.3 (m, 12H) 3.0 (m, 2H), 2.60 (m, 6H), 2.3 (s, 2H), 1.9 (s, 3H), 1.7 (m, 1H), 1.4 (m, 1H).

¹H NMR (D2O) δ 7.9 (d, 1H), 7.8 (d, 1H), 7.75 (s, 1H), 7.2 (d, 2H), 7.1 (d, 2H) 4.8 (m, 2H), 3.7 (s, 8H), 3.5 (s, 4H), 3.0 (m, 2H), 2.7 (m, 6H), 2.3 (t, 2H), 1.9 (s, 3H), 1.3 (m, 1H), 1.1 (m, 1H).

The solid is redissolved in 2.6 litres of EtOH and 1.4 litres of monobasic sodium phosphate buffer. The pH is adjusted to a value of 6 by addition of 1N HCl. Finally, 384 g of enzyme derivative obtained from free lipase enzyme from *Aspergillus niger* and immobilised on epoxy resins are added. The reaction is performed in such a manner as to maintain a constant pH and is monitored by means of HPLC. The organic phase is evaporated down to a small volume, is extracted with ethyl acetate in a basic environment and the phases are separated. The upper phase is concentrated until 190 g of a yellow oil are obtained. The yellow oil is resuspended with alcohols. The mixture is refluxed for approx. 30 minutes and is then left to cool, before being filtered and washed. 65 g of (-)4-(4-dimethylamino)-1-(4'-fluorophenyl)-1-(hydroxybutyl)-3-(acyloxymethyl)benzonitrile hydrochloride of a purity of 95.9% are obtained.

### Example 4: Synthesis of (±)4-(4-dimethylamino)-1-(4'-fluorophenyl)-1-(hydroxybutyl)-3-(hydroxymethyl)benzonitrile

The crystallisation mother liquor originating from the preceding step predominantly containing the enantiomer (+)4-(4-dimethylamino)-1-(4'-fluorophenyl)-1-(hydroxybutyl)-3-(acyloxymethyl)benzonitrile is evaporated and then subjected to acid hydrolysis by addition of 300 ml of 1N HCl. The mixture is evaporated and 70 g of solid are obtained which correspond to (±)4-(4-dimethylamino)-1-(4-fluorophenyl)-1-(hydroxybutyl)-3-(hydroxymethyl)benzonitrile, which is recycled and acetylated using the method stated in Example 3.

### Example 5: Synthesis of (-)4-(4-dimethylamino)-1-(4'-fluorophenyl)-1-(hydroxybutyl)-3-(hydroxymethyl)benzonitrile

7 g of (-)4-(4-dimethylamino)-1-(4'-fluorophenyl)-1-(hydroxybutyl)-3-(hydroxymethyl)benzonitrile hydrochloride and 140 ml of methanol are introduced into a 1 litre four-necked flask. 280 ml of 28% ammonia are introduced and the mixture is stirred for approx. 3 hours. The solution is evaporated under a vacuum at 40°C until a residue is obtained. 7 g of (-)4-(4-dimethylamino)-1-(4'-fluorophenyl)-1-(hydroxybutyl)-3-(hydroxymethyl)benzonitrile are obtained in the form of a colourless oil.

### Example 6: Synthesis of s-citalopram

7 g of (-)4-(4-dimethylamino)-1-(4'-fluorophenyl)-1-(hydroxybutyl)-3-(hydroxymethyl)benzonitrile in the form of a colourless oil and 75 ml of toluene are introduced into a 500 ml four-necked flask under an inert nitrogen atmosphere. 1.8 ml of mesyl chloride are introduced at a temperature of 0°C, an interval of approx. 10 minutes is left and 9.5 ml of triethylamine are added dropwise. The reaction mixture is stirred at 0°C for approx. 2 h. The reaction is stopped with 30 ml of water The phases are separated. The upper phase is evaporated. 4.6 g of oil are obtained.

The oil is resuspended with 10 ml of acetone and salified with 1.5 g of anhydrous oxalic acid. The mixture is filtered and washing performed with acetone. The product is placed in an oven at 60°C under a vacuum for 2 hours. 3.8 g of s-citalopram are obtained with a purity of 99.5%, ee=99% [α]_{D}= +12.4.

### Example 7: Synthesis of s-citalopram

2.8 g (0.081 moles) of (-) 4-(4-dimethylamino)-1-(4'-fluorophenyl)-1-(hydroxybutyl)-3-(hydroxymethyl)benzonitrile and 6.43 g of triphenylphosphine (0.0245 moles, equal to 3 moles per mole of starting substrate) dissolved in 100 ml of tetrahydrofuran are added to a four-necked flask under a nitrogen atmosphere at 0°C and the mixture is stirred. 6.8 ml of ethyl azodicarboxylate (0.043 moles, equal to 5.3 moles per mole of starting substrate) dissolved in 20 ml of tetrahydrofuran and 1.86 g sodium tert.-butylate (0.01944 moles) dissolved in 15 ml of tetrahydrofuran are slowly added dropwise. The mixture is stirred overnight, after which the reaction is terminated by dropwise addition of 30 ml of 1N HCl. The mixture is evaporated, 70 ml of toluene and 70 ml of water are added and the pH is adjusted to 9.4 by addition of aqueous ammonia. The phases are separated and the organic phase is evaporated under a vacuum. An oil (2.2 g) is obtained, to which are added 5 ml of acetone; 0.94 g of oxalic acid are added, the mixture is filtered, giving rise to 2 g of escitalopram oxalate with [α]_{D}= +12.4.

NOTE 1: Once addition of the mixture of the 2 Grignard reagents to the suspension of 5-cyanophthalide is complete, an aliquot of the reaction is subjected to NMR analysis (BRUKER AMX 3-400).

| ¹H-NMR (DMSO-d6) | | | |
|---|---|---|---|
| δ(ppm) | Multiplicity | No. H | Attribution |
| 7.83-7.70 | m | 3 | H4, H5, H6 |
| 7.22-7.05 | m | 4 | H7, H8 |
| 5.1 | s | 1 | H2 |
| 4.52-4.48 | d | 1 | H1 |
| 4.00-3.96 | d | 1 | H1' |
| 2.98-2.16 | m | 6 | H9, H10, H11 |
| 1.69 | s | 6 | H12 |

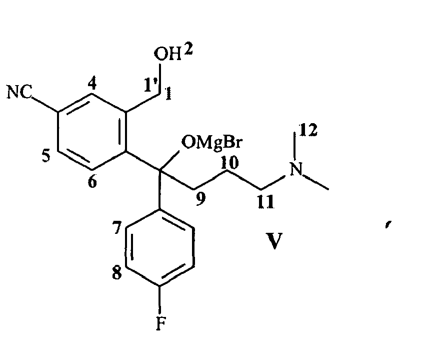

This ¹H-NMR is compared with the compound having the two free hydroxyl groups shown below. This comparison reveals that the signal at 6.50 ppm, which corresponds to the hydroxyl in position 3, is absent in the intermediate I.

| ¹H-NMR (DMSO-d6) | | | |
|---|---|---|---|
| δ(ppm) | Multiplicity | No. H | Attribution |
| 7.89-7.74 | m | 3 | H4, H5, H6 |
| 7.27-7.07 | m | 4 | H7, H8 |
| 6.50 | s | 1 | H3 |
| 5.16 | s | 1 | H2 |
| 4.60-4.56 | d | 1 | H1 |
| 4.08-4.04 | d | 1 | H1' |
| 2.29-2.23 | m | 2 | H9 |
| 2.16-2.11 | m | 2 | H11 |
| 2.01 | s | 6 | H12 |
| 1.39-1.19 | m | 2 | H10 |

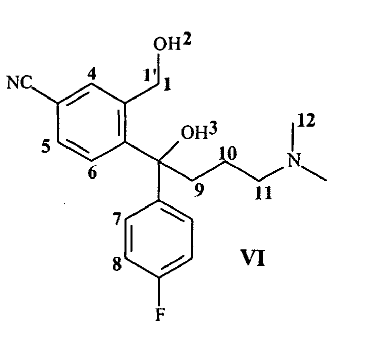

## Claims

1. A process for the preparation of escitalopram comprising the reaction of a complex of the formula IV, 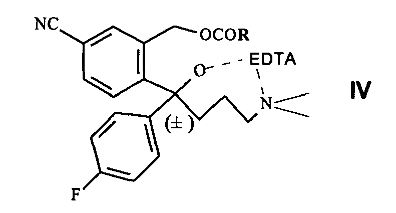 where R represents a C₁-C₄ alkyl residue or an aryl residue, with an esterase and the subsequent treatment thereof in a basic environment to yield the compound of the formula II

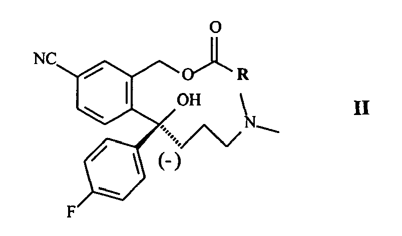

2. A process according to claim 1, **characterised in that** said esterase is an esterase from *Aspergillus niger.*

3. A process according to any one of the preceding claims, **characterised in that** R is methyl.

4. A process according to any one of the preceding claims, **characterised in that** said reaction is performed in a mixture of alcohol and water.

5. A process according to any one of the preceding claims, **characterised in that** said alcohol is a C₁-C₄ alcohol, preferably ethanol.

6. A process according to any one of the preceding claims, **characterised in that** said mixture has a ratio of between 0.2-1.2 volumes of water and 1.0-1.5 volumes of alcohol, preferably 0.7 volumes of water and 1.3 volumes of alcohol.

7. A process according to any one of the preceding claims, **characterised in that** said reaction is performed at a temperature of between 15 and 35°C, preferably between 20 and 25°C.

8. A process according to any one of the preceding claims, **characterised in that** said mixture of alcohol and water is used in an amount of 3-5 litres, preferably 3.5-4 litres, per mole of complex of the formula IV.

9. A process according to claims 4-8, **characterised in that** the water is introduced in the form of phosphate buffer, preferentially monobasic potassium phosphate buffer.

10. A process according to any one of the preceding claims, **characterised in that** said esterase is immobilised on resin, preferably epoxy resin.

11. A process according to any one of the preceding claims, **characterised in that** said esterase is used in an amount of 2500-3200 units, preferably 2800-2900 units, per mole of complex of the formula IV.

12. A process according to any one of the preceding claims, **characterised in that** said treatment in a basic environment proceeds at a pH of between 7 and 9.5.

13. A process according to any one of the preceding claims, **characterised in that** the resultant compound II is converted by hydrolysis into the compound of the formula III 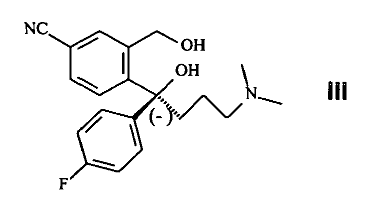 which is converted into escitalopram by condensing the two hydroxyl groups.

14. A process according to claim 13, **characterised in that** said hydrolysis is basic.

15. A process according to any one of the preceding claims, **characterised in that** the (+)4-(4-dimethylamino)-1-(4'-fluorophenyl)-1-(hydroxybutyl)-3-(acyloxymethyl)benzonitrile is racemised and recirculated into the reaction with the esterase.

16. A process according to claim 15, **characterised in that** said racemisation proceeds in an acidic environment, preferably at a pH of between 0.5 and 4.

17. A complex of the formula IV, 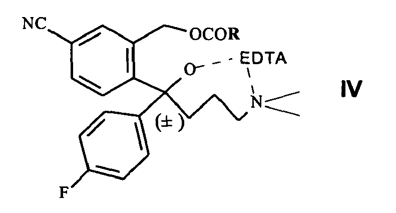 where R represents a C₁-C₄ alkyl residue or an aryl residue.

18. A complex according to claim 17, where R is methyl.

19. Use of a complex according to claims 17-18 in the preparation of escitalopram.
